Europäisches Patentamt

European Patent Office

Office européen des brevets

(19)

(11) Publication number: **0 441 483 A2**

(12) **EUROPEAN PATENT APPLICATION**

(21) Application number: **91300287.9**

(22) Date of filing: **15.01.91**

(51) Int. Cl.$^5$: **C12N 15/12, C12N 15/81, C12N 1/19, G01N 33/58, G01N 33/68, C12Q 1/68, C12Q 1/34, C12Q 1/48**

(30) Priority: **16.01.90 US 464837**
**09.01.91 US 639506**

(43) Date of publication of application:
**14.08.91 Bulletin 91/33**

(84) Designated Contracting States:
**AT BE CH DE DK ES FR GB IT LI NL SE**

(71) Applicant: **BAYLOR COLLEGE OF MEDICINE**
**One Baylor Plaza**
**Houston, TX 77030 (US)**

(72) Inventor: **McDonnell, Donald P.**
**1246 Manchester Circle**
**Missouri City, Texas 77459 (US)**
Inventor: **O'Malley, Bert W.**
**629 Ramblewood**
**Houston, Texas 77079 (US)**
Inventor: **Conneely, Orla M.**
**7350 Kirby Drive 17**
**Houston, Texas 77030 (US)**

(74) Representative: **Adams, William Gordon et al**
**RAWORTH, MOSS & COOK 36 Sydenham Road**
**Croydon Surrey CR0 2EF (GB)**

(54) **Expression vectors that produce steroid receptors, steroid receptor chimera, screening assays for steroid receptors and clinical assays using synthesized receptors and receptor vectors.**

(57)    The present invention relates to a method for making an expression vector which produces a biological active receptor. The method includes synthesizing a linker having the carboxyl six amino acids of ubiquitin and an internal restriction site and cloning the synthesized linker to form a plasmid. The plasmid is then digested with a restriction endonuclease which is compatible with the internal restriction site in the synthesized linker. After the digestion of the plasmid a cDNA coding for the receptor is inserted into the compatible restriction site and the plasmid is then used to transform yeast. In the procedure, the yeast that is usually used is from the strains of Saccharomyce and Aspergillus. In addition, numerous vectors which express the human vitamin D receptor, human androgen receptor, human progesterone receptor, thyroid receptor, retinoic acid receptor, chicken progesterone receptor, chicken vitamin D receptor, rat vitamin D receptor and COUP protein are described. A further aspect of the invention is the reporter vector which includes the proximal promoter element of the yeast iso-l-cytochrome C and a structural gene selected from E. coli either the õ-Galactosidase gene or the Galactokinase gene. The reporter vector is used to detect for agonist and antagonists for the steroid hormone receptor. A further element of the invention is a chimera made up of the DNA binding domain from one receptor and the hormone binding domain from another receptor. The chimera is used in an assay to identify the function of the unknown receptor component. Additional aspects are clinical and laboratory assays for biological compounds using the receptors synthesized from the expression vectors and using receptor vectors.

EP 0 441 483 A2

Figure 1

# EXPRESSION VECTORS THAT PRODUCE STEROID RECEPTORS, STEROID RECEPTOR CHIMERA, SCREENING ASSAYS FOR STEROID RECEPTORS AND CLINICAL ASSAYS USING SYNTHESIZED RECEPTORS AND RECEPTOR VECTORS

## Field of the Invention

The present invention relates generally to steroid hormone receptors, screening assays for steroid receptor ligands, chimeric receptors and assays for determining the amount and efficiency of steroid receptor binding, the identity of unknown receptors and agonists and antagonists of receptors and assays using synthesized receptors or receptor vectors.

## Background of the Invention

Steroid hormones are potent modulators of transcriptional events that together regulate the complex processes associated with differentiation, homeostasis and development. The mechanism of action of these molecules is related in that the effector molecule binds to a specific intracellular receptor. Such binding alters the structure of the receptor, increasing its affinity for specific recognition sites within the regulatory region of target genes. In this way the steroid directs a program of events that leads to a change in cell phenotype.

Several members of the steroid receptor family have been cloned and their functional domains have been genetically dissected. The functional domains include a highly conserved DNA binding domain, a hormone binding domain spanning the majority of the carboxyl terminus of the protein, and an amino terminal region of varying length that plays a role in modulation of the specificity of the receptor. This domain organization demonstrates a modular organization for the receptors, each comprising separate functional entities. This interpretation was further strengthened by showing that the DNA binding region of the estrogen receptor could be replaced with the DNA binding region of the glucocorticoid receptor. This chimeric receptor regulates glucocorticoid responsive genes in an estrogen dependent manner. Furthermore, the molecules do not have to be related, for example the DNA binding region of the Yeast Gal4 protein was fused to the estrogen receptor and functioned as a steroid dependent transcription factor.

The DNA binding domain has been mapped precisely and is limited to a 66 amino acid region within the receptor. This region is very highly conserved at the amino acid level and typically the receptors demonstrate 40-50% relatedness. More remarkable than the overall amino acid similarity is the positional conservation of nine invariant cysteines in this region. So remarkable is this conservation that it allows classification of molecules containing this cysteine motif as members of the steroid receptor superfamily without direct proof that the protein is a transcription factor or even binds ligand.

Using a DNA sequence containing the conserved DNA binding domain of the receptors, several groups have identified clones corresponding to other members of this superfamily. Ligands for only two of these molecules have been identified, thyroid hormone and retinoic acid.

## SUMMARY OF THE INVENTION

An object of the present invention is the development of expression vectors that produce steroid hormone receptors.

An additional object of the present invention is the construction of a series of reporter vectors that are used to assay transcriptional activity of receptor expression vectors.

A further object of the present invention is the development of a series of chimeric receptor expression vectors.

An additional object of the present invention is the development of an assay for measuring agonists and antagonists to steroid ligands.

Another object of the present invention is development of an assay to identify ligands of novel steroid receptors.

An additional object of the present invention is an improved radioreceptor assay for detecting biological compounds in a biological sample.

A further object of the present invention is a sensitive assay for biological compounds in a complex matrix by using reporter systems.

Thus in accomplishing the foregoing objects, there are provided in accordance with one aspect of the present invention a method of making an expression vector which produces a biologically active receptor, comprising the steps of : synthesizing the linker having the carboxyl six amino acids of ubiquitin and an internal restriction site ; cloning said synthetic linker to form a plasmid ; digesting said plasmid with a restriction endonuclease compatible with the internal restriction site ; inserting a cDNA coding for a receptor into the restriction site of said plasmid ; and transforming yeast with said receptor cDNA.

In more particular embodiments of this invention the yeast is selected from the group consisting of Saccharomyces and Aspergillus and the steroid receptor cDNA can be selected from the group of cDNA's coding for estrogen receptor, androgen receptor, vitamin D receptor and progesterone receptor.

An additional aspect of the present invention is a reporter vector comprising a proximal promotor element of yeast iso-l-cytochrome C ; and a structural gene fused to said promotor wherein said structural gene is selected from the group consisting of β-Galactosidase and Galactokinase.

A further aspect of the present invention is a receptor agonist or antagonist transcription assay comprising the steps of : growing a yeast strain in appropriate media ; adding the test agonist or antagonist ; and examining the resulting growth.

In preferred embodiments the receptor vector includes a β-Galactosidase as the structural gene and the media can include 5-bromo-4-chloro-3-indolyl β-D-Galactopyranoside, β-Galactosidase substrate, $CuSO_4$ and agar or Galactokinase as the structural gene and the media can include about 3% glycerol, about 2% ethanol, agar, $CuSO_4$ and about 0.1% 2-deoxy-D-galactose.

An additional embodiment of the invention is a method of making a chimera receptor comprising the steps of : excising with a restriction endonuclease a DNA binding domain from a known receptor ; introducing a compatible restriction site at a DNA binding domain in an unknown receptor ; excising the DNA binding domain from the unknown receptor at said compatible restriction site ; and inserting into said unknown receptor at the compatible restriction site the known DNA binding domain.

Other and further objects, features and advantages will be apparent in the following description of presently preferred embodiments of the invention given for the purposes of disclosure when taken in conjunction with the accompanying drawings.

## DESCRIPTION OF THE DRAWINGS.

Figure 1 is a schematic of the strategy used to produce biologically active steroid receptors in yeast. Indicated are the locations of the CUPI promoter, CYCI terminator, TRPI selection marker, 2 μb sequences and the ubiquitin molecule. All cDNAs were inserted into the AFLII-KPNI sites of the expression vector.

Figure 2 shows the strategy used to produce unfused steroid receptors in yeast. Indicated are the locations of the CUPI promoter, CYCI terminator, TRPI selection marker and 2 μb sequences. cDNAs are inserted into the NcoI-KpnI sites of the expression vector.

Figure 3 shows the production of a recombinant vitamin D receptor in yeast as seen by Western immunoblotting.

Figure 4 shows the analysis of the functional properties of the recombinant receptor. Cytosol containing the recombinant receptor was analyzed by saturation (A) and Scatchard analysis (B). Solid circles represent total binding, open circles specific binding and open squares nonspecific binding. The DNA binding properties of the receptor were analyzed by calf thymus DNA cellulose chromatography (C). Open circles represent the elution profile of tritiated hormone/receptor complex. The solid line represents the KCl gradient used to elute the bound receptor.

Figure 5 is a schematic of the two types of reporter vectors used to assay the transcriptional activity of the receptor. The YRP(A) plasmid contains the proximal promoter elements of the yeast iso-l-cytochrome C gene fused to the structural gene for E. coli β-Galactosidase. The YRP(B) plasmid has the same promoter elements but it was fused to the structural gene for E. coli Galactokinase. In each case, the site of insertion of the steroid receptor response elements is also included.

Figure 6 is a schematic of the strategy employed to isolate steroid receptor agonists or antagonists using the β-Galactosidase transcription assay.

Figure 7 is a schematic of the strategy employed to isolate steroid receptor agonists or antagonists using the Galactokinase transcription assay.

Figure 8 is a schematic of the strategy used to convert the progesterone receptor into a chimera.

Figure 9 shows the strategy used to convert the human estrogen receptor into a superactive receptor.

Figure 10 shows the transcriptional activity of GAL4 in the presence of the estrogen receptor on the YRpEG3 reporter.

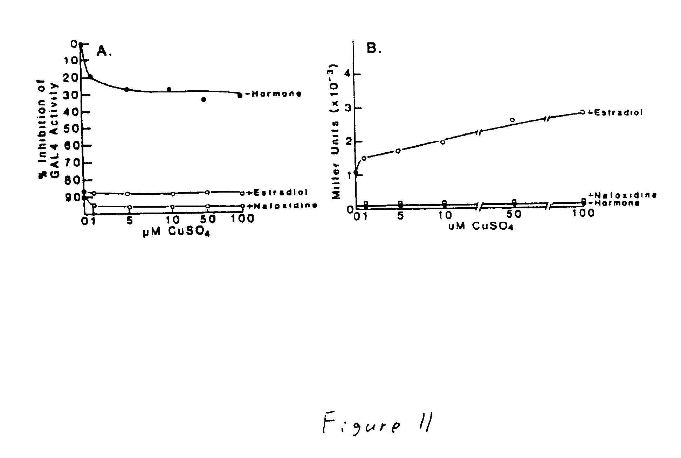

Figure 11 shows the transcriptional activity of GAL4 on the presence of low concenetrations of estrogen receptor on the YRpEG3 reporter.

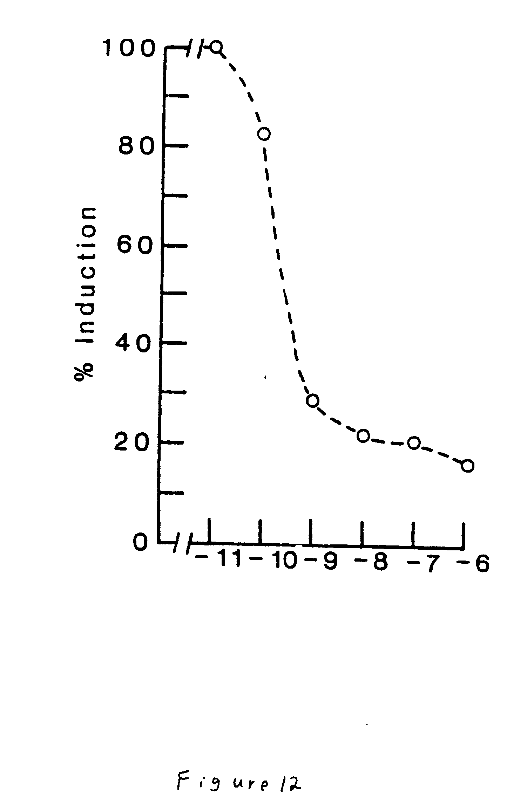

Figure 12 shows inhibition of GAL4 mediated induction of transcription.

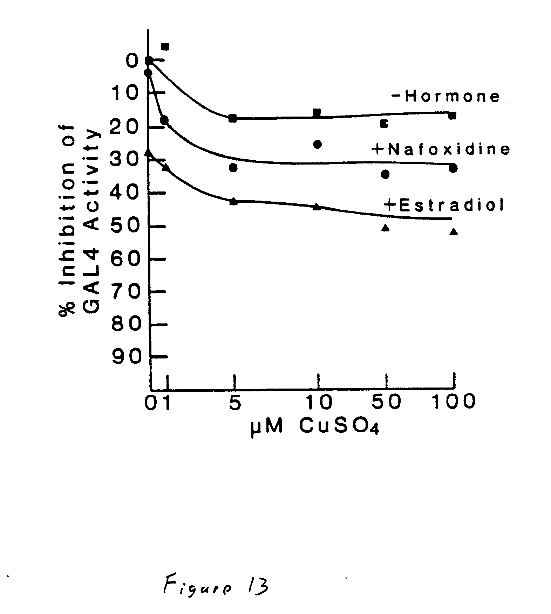

Figure 13 shows transcriptional activity of GAL4 in low concentrations of estrogen receptor on the YRpHSEREG4 reporter.

The drawings and figures are not necessarily to scale and certain features of the invention may be exaggerated in scale or shown in schematic form in the interest of clarity and conciseness.

## DETAILED DESCRIPTION OF INVENTION

It will be readily apparent to one skilled in the art that various substitutions and modifications may be made to the invention disclosed herein without departing from the scope and spirit of the invention.

The term restriction endonuclease as used herein defines an enzyme that cleaves DNA at sequence specific sites, thereby creating double stranded breaks.

The term "restriction site" as used herein refers to the sequence specific cleavage sites of a restriction endonuclease.

The term "compatible restriction site" as used herein defines a specific cleavage site of restriction endonuclease which is the same as that of the molecule which is to be inserted. For example, if the molecule to be inserted has an EcoRI restriction site, the compatible restriction site in the vector would be an EcoRI site. One skilled in the art will readily recognize that there are multiple compatible restriction sites depending on which restriction enzyme is used.

The term "transforming" as used herein describes a mechanism of genetic transfer whereby DNA extracted from a donor is able to induce permanent genetic changes in a recipient with respect to those characteristics in which the donor and recipient differ. For example, using an expression vector to incorporate genes present in the expression vector into strains of yeast.

The term "chimera" as used herein means a strand of DNA formed by the fusion of the genetic sequence of two different receptors. For example, steroid receptors are composed of both a DNA binding domain and a hormone binding domain, in the chimera receptor the DNA binding domain is composed of the genetic code from one receptor and the hormone binding domain is composed of the genetic code from another receptor. These two domains are fused to form a chimera receptor. The domains can be from different species or from different receptors within a species.

The term "superactive receptor" as used herein means a modified receptor that has an increased ability to activate transcription.

The term "filling in" as used herein describes the activity of the enzymes T4 DNA polymerase or E. coli DNA polymerase when used to repair the damaged or protruding 3′ or 5′ ends of DNA.

The term "Miller units" as used herein is a relative measure of levels and is calculated relative to a control. It is a measure of the effect on transcriptional activity of receptors or the potency of a particular against or antagonist.

Mathematically :

$$\text{Miller units} = \frac{(O.D._{420} \times 1000)}{(t_{min})} \times \text{Mg protein assayed}$$

The term "biological specimen" as used herein means a tissue or fluid collected from a human or non-human organism. For example it can be a clinical sample such as blood, tears, sweat, urine, fecal matter, tissue biopsy or spinal fluid. It can be a laboratory sample such as tissue culture fluid or growth medium. One skilled in the art will readily recognize that a variety of specimens can be used.

The term "label" as used herein means a chemical or element which has been attached to a molecule to help identify the molecule in a mixture. For example labels can include radioisotopes, fluorescers, chemiluminescers, enzymes and antibodies.

The term "biological compound" as used herein means the chemical compound of interest. For example a hormone, a steroid, a vitamin, etc.

## Development of Expression Vectors

A series of expression vectors have been developed to express mammalian steroid receptors in yeast. The receptor is produced in yeast by ubiquitin fusion. The ubiquitin molecule is co-translationally removed by a host processing enzyme, thus leaving an unfused recombinant receptor.

The method of making an expression vector which produces a biologically active receptor, comprises the steps of : synthesizing a linker having the carboxyl 6 amino acids of ubiquitin and an internal restriction site ;

cloning said synthesized linker to form a plasmid ; digesting said plasmid with a restriction endonuclease compatible with said internal restriction site ; inserting a cDNA coding for a receptor into the compatible restriction site of said plasmid and transforming yeast with said receptor cDNA plasmid. Upon growing the yeast, the specific receptor will be synthesized. One skilled in the art can readily isolate and purify the synthesized receptors using standard biochemical techniques.

Using this method, expression vectors for human estrogen receptors, androgen receptors, progesterone receptors, and vitamin D receptors have been developed. Expression vectors containing human, chicken or rat receptors have been developed.

Specific embodiments of expression vectors which have been made include YEpV1 for expression of human vitamin D receptor, YEpE2 for expression of human estrogen receptor, YEpAR1 for expression of human androgen receptor, YEpP2 for expression of chicken progesterone receptor ($\beta$ form), YEpP8 for expression of chicken progesterone (A form) ; YEpHPR 1 for expression of human progesterone (A form), YEpHPR2 for expression of human progesterone (B form), YEpHTR1 for expression of thyroid receptor ($\beta$ form), YEpHTR2 for expression of thyroid receptor ($\alpha$ form), YEpRAR for expression of retinoic acid receptor, YEpCOUP for expression of the COUP protein, YEpV9 for expression of chicken vitamin D receptor and YEpV10 for expression of rat vitamin D.

The biological-biochemical properties of the receptors produced by the expression vectors appear to be identical to the properties described for the native or wild-type receptors.

Although a variety of yeast strains can be transformed, in the preferred embodiment Saccharomyce and Aspergillus are used.

## Reconstitution of Steroid

## Responsive Transcription Units

A series of reporter vectors (plasmids) were constructed in order to assay the transcriptional activity of the receptors in yeast. These individual reconstituted transcription units are useful to screen and evaluate compounds for their steroid agonistic or antagonistic activity. Additionally, the reconstituted transcription units can be used to identify processes that can convert steroid precursors into active compounds.

The reporter vector is comprised of the yeast iso-l-cytochrome C proximal promoter element fused to a structural gene, wherein said structural gene is selected from the group consisting of $\beta$-Galactosidase, Galactokinase and URA3.

In the preferred embodiment the vector is further comprised of an insertion site for a receptor response element. An additional embodiment includes a receptor response element.

The vectors which include $\beta$-Galactosidase as an indicator of transcriptional activity are derived from the parent vector pC2 while the vectors which include Galactokinase are derived from YCpR1 vector. In the preferred embodiment, the structural genes originate from E. coli.

## Agonist and Antagonist Transcription Assay

An additional embodiment of the present invention includes a receptor agonist or antagonist transcription assay, comprising the steps of : growing a reporter vector in appropriate medium ; adding a test agonist or antagonist ; and examining the resulting growth. The reporter vector can be based on a $\beta$-Galactosidase, Galactokinase or an URA3 detection system. The results obtained depend on what detection system is used. For instance, if the Galactokinase structural gene is used, the presence or absence of growth is important in determining whether an agonist or antagonist is present. The appropriate hormone by itself leads to cell death. On the other hand if the $\beta$-Galactosidase transcription assay is used, the results are measured by a color change. In the absence of hormone the colonies appear white. If a hormone is present, the colonies appear blue. If an URA3 detection system is used, the cells are killed in the presence of 5-Flouro-orotic acid. Thus, depending on whether the Galactokinase, $\beta$-Galactosidase or URA3 system is used, this assay system can determine the presence of, absence of, efficiency of and effectiveness of agonists and antagonists by examining the growth or color of the colonies.

## Chimera Receptors

The method of making a chimera receptor comprises the steps of : excising with a restriction endonuclease a DNA binding domain from a known receptor ; introducing a compatible restriction site at a DNA binding domain in an unknown receptor ; excising the DNA binding domain from the unknown receptor at said compatible restriction site ; and inserting into said unknown receptor at the compatible restriction site the known DNA binding domain.

If the known DNA binding domain does not have an internal restriction site the method can include the further step of introducing a restriction site into the known DNA binding domain prior to excising the known DNA binding domain from the receptor. The restriction site which is introduced into the unknown DNA binding domain must be compatible to the internal restriction site in the known DNA binding domain or with the introduced restriction site. Thus, when the DNA binding domain is excised from the unknown receptor, the known binding domain can be inserted because of the compatible restriction site.

Once the chimera receptor is made, it can be used in an assay for the identification of unknown receptor ligands. The assay for identification for unknown receptor ligands comprises the steps of : growing the chimera receptor ; adding appropriate agonists to screen the unknown portion of the chimera receptor ; and measuring the response of the known DNA binding domain. The DNA binding domain response will depend on the amount of putative agonist which binds to the receptor. If no binding occurs, there is no response, whereas if the agonist binds, the DNA binding domain should quantitively respond. Thus, this assay can be used to screen a wide variety of chemicals to determine the sensitivity and selectivity of the unknown ligands.

A second type of chimera has also been developed, allowing the production of a superactive receptor. In this case, the defined activating domains of the viral VP16 protein is inserted into $NH_2$ terminus of the wild type receptor.

Another aspect of the present invention is an improvement in the assay for biological compounds. The assay to measure a biological compound in a biological specimen comprises the steps of pre-labeling a biologically active receptor with an appropriate labeled biological compound, wherein the receptor is synthesized from the previously described expression vector ; adding a sample of the specimen to the pre-labeled receptor ; and measuring the amount of labeled biological compound displaced by the biological compound in the specimen to determine the amount of biological compound in the specimen.

In the preferred assay the label is a radioisotope.

A further embodiment of the present invention is a kit for measuring a biological compound in a biological specimen, comprising, a container having a biologically active receptor synthesized from the previously described expression vector. The kit can further include a radio-labeled compound for binding to the biologically active receptor. In another embodiment the kit further includes control samples to generate a standard curve.

An additional embodiment of the present invention is an assay for biological compounds using the receptor and the previously described reporter vector. The assay for measuring a biological compound in a biological specimen comprises the steps of growing yeast cells containing a biologically active receptor and a reporter vector, wherein the reporter vector comprises a proximal promoter element of yeast iso-l-cytochrome fused to a structural gene selected from the group consisting of β-Galactosidase and Galactokinase ; adding a sample of the specimen to the yeast cells ; and determining the amount of biological compound in the specimen by measuring the activity of the protein product of the structural gene.

In specific embodiments the receptor is an estrogen receptor and the structural gene is β-Galactosidase.

Other specific embodiments of the assay include using a chimeric receptor or a superactive receptor. The superactive receptor includes an estrogen receptor with an activation domain of a herpes simplex VP16 protein included within its reading frame. The chimeric receptor preferably includes an estrogen response element and a non-estrogen ligand binding domain. The binding domain is specific to the biological compound of interest.

In addition to the previously described receptors either the improved assay or the receptor assay can also be used with cortisone, prednisone, prednisolone and aldosterone receptors.

Another embodiment of the present invention is a reporter for detecting DNA binding, comprising a sequence for any transcription factor that binds to DNA and a sequence for a response element from any biologically active receptor. The two sequences (transcription factor and response element) are fused such that the proteins which bind to the factor and element cannot occupy the fused sequence at the same time. Thus, the sequences must be close together. In the preferred embodiment, at least one nucleotide base from the transcription factor sequence overlaps with the response element sequence. Thus, when this reporter is used in an assay either the ligand for the transcription factor or the ligand for the response element can bind, but both cannot simultaneously bind. Although any transcription factor that binds DNA and any response element can be used, one preferred embodiment includes the binding protein response factor selected from the group consisting of GAL4, GCN4, yAP1, and ACE1 ; and the response element selected from the group consisting of recognition sequences for estrogen receptor, androgen receptor, vitamin D receptor, progesterone receptor, cortisone receptor, prednisone receptor, prednisolone receptor, aldosterone receptor, retinoic acid receptor and thyroid receptor.

In an assay for a biological compound the fused sequence reporter and a biologically active receptor are inserted into cells. For example, Saccharomyce or Aspergillus. The cells are grown in an appropriate media. The biological compound to be tested is added to the growing cells. Then the amount of transcriptional activity is measured. The amount of transcriptional activity is proportional to the amount of biological compound in the biological specimen.

By measuring the amount of transcription or inhibition of transcription, the assay can be further expanded to detect agonists and antagonists of the active receptor and/or the protein binding the transcription factor.

The assay can be used to detect and find known or unknown transcription factors and biologically active receptors.

One embodiment is a method of identifying unknown transcription factor. In this method a known biologically active receptor and a reporter which includes the unknown transcription factor fused to a known response element are inserted into a cell. The cell is grown in appropriate media. Next, the compound that binds to the biologically active receptor is added and test protein is added to identify the transcription factor. The resultant transcriptional activity is examined to determine the identity of the unknown transcription factor.

Another embodiment is a method of identifying unknown receptor. In this embodiment the unknown receptor and a reporter which includes a known transcription factor and the response element of the unknown receptor are added to a cell. The cell is grown in appropriate media. A test compound is added to the growing cells. to identify the biologically active receptor. The resultant transcriptional activity is examined to identify the receptor.

The following examples are offered by way of illustration and are not intended to limit the invention in any manner. In these examples, all percentages are by weight if for solids and by volume for liquids and all temperatures are in degrees Celsius unless otherwise noted.

## EXAMPLE 1

### Construction of the Expression Vector YEpV1 and its Biological-Biochemical Properties.

The expression vector YEpV1 directs the synthesis of the human vitamin D receptor and was constructed as follows : A synthetic linker was synthesized which included the carboxyl six amino acids of ubiquitin and contained an internal EcoR1 site. This linker was cloned into the EcoR1-Kpn1 site of pGEM4. The resultant plasmid pG42 was digested with EcoR1 and the 2.0 Kb EcoR1-EcoR1 fragment of the vitamin D receptor cDNA was inserted into that site. In the correct orientation this yielded the plasmid BCpV1, containing an inframe fusion of ubiquitin to the receptor. Following amplification, the Af111-Kpn1 fragment was excised and subcloned into the corresponding sites of YEp42. The resultant plasmid was called YEpV1. This plasmid was amplified, purified and used to transform Saccharomyces cerevisae using lithium acetate transformation procedures. Transformants were selected by tryptophan auxotrophy.

Recombinant receptor was produced by growing the transformants overnight in minimal media supplemented with 2% glucose. The following day the cells were diluted 10-fold and allowed to grow to an optical density of 1.0 at 600nM. Production of the receptor was initiated by the addition of 100µM $CuSO_4$. The cells were allowed to grow for an additional two hours, harvested by centrifugation and washed twice with ice cold TKO (10mM Tris-HCl pH 7.5, 5mM DTT). The final pellet was resuspended in 450µl of TKO and the cells were disrupted by vortexing three times for 1 minute in a microfuge tube that contained 1/3 volume of glass beads. A fourth vortexing was done following the addition of 50µl of 3M KCl to ensure extraction of the receptor from the nucleus. The cellular debris was removed by centrifugation at 12,000g for 10 minutes. The supernatant was recovered and the total protein was measured. Cytosols were analyzed immediately.

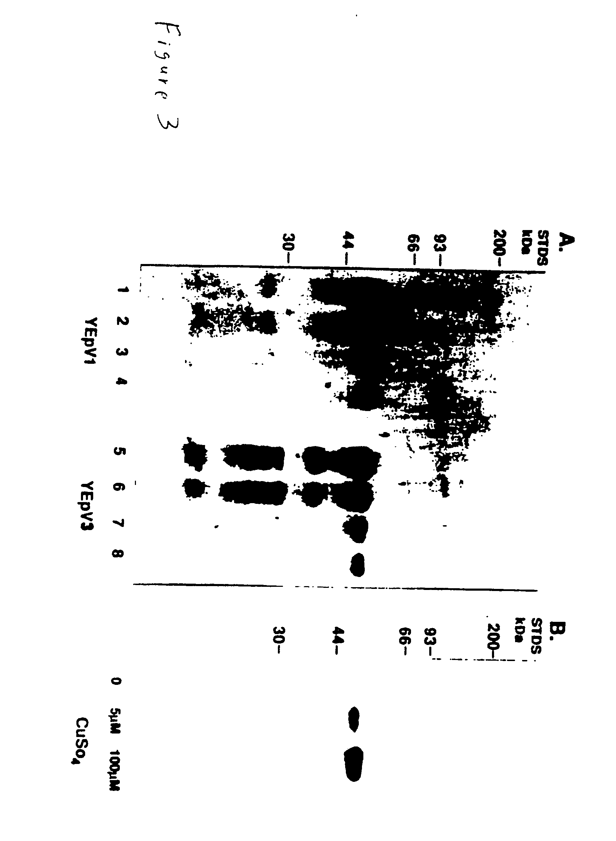

Saccharomyces cerevisae strain F762 was transformed with YEpV1 and the receptor synthesis was examined under conditions where 0, 5 and 100 µM $CuSO_4$ was added to the media. Cytosols from the recombinant receptor was analyzed by Western immunoblotting using a monoclonal antibody specific for the vitamin D receptor (Fig. 3). Lanes 1, 2, 3, 4 represent 10 µG and 1 µG of total protein extracted from two independent clones transferred with YEpV1. The inducibility of the receptor in the system was quantified by immunoblot. In the absence of $CuSO_4$ (uninduced cells) no immunoreactive material corresponding to receptor was detectable. However, upon the addition of increasing amounts of $CuSO_4$ the human vitamin D receptor was detected as an intact protein species with a molecular mass of 47kDa.

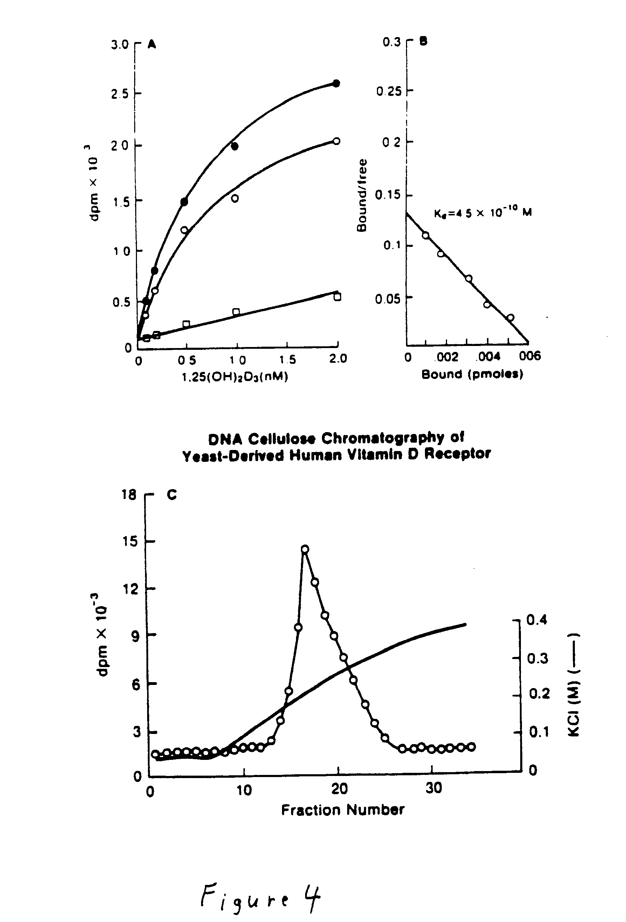

An analysis of the hormone binding properties of the recombinant receptor is shown in Fig. 4. In cytosol from cells transformed with the receptor expression plasmid, a high affinity saturable binding site was detected (Fig. 4A). Cytosols from non-transformed cells, i.e. wild-type, do not show specific binding of vitamin D. The affinity of this species is similar to that observed by others for the wild-type mammalian receptor and similar to current estimates of the affinity of the mammalian receptor. The DNA binding properties of the receptor were examined using calf thymus DNA cellulose chromatography. Cytosol was labeled with tritiated hormone and loaded onto a DNA cellulose column (Fig. 4B). Following extensive washing with low salt buffer the bound proteins were eluted with a linear gradient of KCl. The profile shown demonstrates that the peak of radioactivity elutes at 0.22M KC1 consistent with the wild-type receptor. Thus the biochemical properties of the recombinant receptor are identical to the wild-type receptor.

## EXAMPLE 2

### Construction of an Unfused Steroid Receptor

Alternatively the steroid receptor can be produced in an unfused form. The unfused receptor employs either the wild type ATG, or, as in the case of the estrogen receptor, an altered DNA sequence which retains the amino acid sequence, but allows more efficient translation initiation.

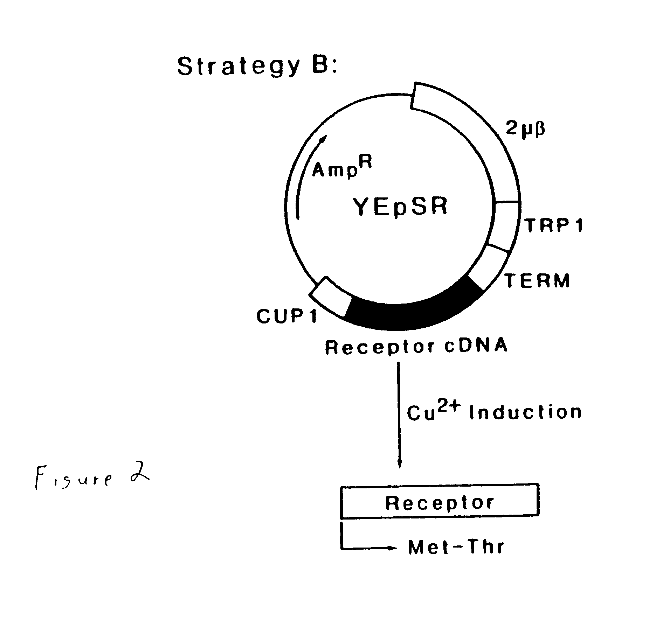

The human estrogen receptor cDNA was inserted into an EcoR1 site of pGEM 3 such that the 5' end of the cDNA was adjacent to the SP6 promoter. The 5' untranslated region was removed by digestion of this plasmid with Tth111 and BamH1. An oligonucleotide containing an Ncol site and the sequences required for optimal translation initiation was inserted into the plasmid. The Nco1-EcoR1 fragment of this plasmid was purified, filled in with T4 polymerase and blunt end ligated into the cut and filled EcoR1 site of the vector RC4. This plasmid is a low copy plasmid containing the CUP1 promoter and CYC1 termination sequences. Furthermore it contains the TRP1 ARS and CEN 3 sequences that allow propagation of the plasmid in yeast. (Fig. 2).

High copy equivalents of this vector were constructed by purifying the BamH1 - Cla1 fragment of this plasmid, filling in the ends with T4 polymerase and inserting the resultant fragment into the Smal phosphatased Yep351 or 352 plasmids.

This vector is modular and allows the insertion of any yeast promoter, selectable marker, or conversion into integrating vectors. Other receptor cDNAs are easily substituted for the estrogen receptor.

The recombinant receptor can be produced by growing the transformants as described in Example 1. Cytosols from the recombinant receptor was analyzed by Western immunoblotting using a monoclonal antibody specific for the vitamin D receptor (Fig. 3). Lanes 5 and 7 and 6 and 8 contain 10 μG and 1 μG of protein from unfused transformations with YEpV3. The inducibility of the receptor in the system was quantified by immunoblot. In the absence of $CuSO_4$ (uninduced cells) no immunoreactive material corresponding to receptor was detectable. However, upon the addition of increasing amounts of $CuSO_4$ the human vitamin D receptor was detected as an intact protein species with a molecular mass of 47kDa.

## EXAMPLE 3

### Comparison of Fused and Unfused Transformants

Cytosol from the transformants of Examples 1 and 2 containing the receptor vectors was analyzed initially by Western immunoblot (Fig. 3A). In this case both vector systems directed approximately the same amount of synthesis. However, it is obvious that the receptor produced as a fusion protein (Fig. 3A, lanes 1-4) is of better quality than that produced in the unfused state (Fig. 3A, lanes 5-8) ; the latter is degraded presumably due to proteolysis. Based upon comparisons with the immunoreactive signal of a known amount of chicken vitamin D receptor we estimate that these strains are producing the receptor equivalent to approximately 0.2% of the total soluble protein or about 150,000 molecules per cell. This represents a level 50-100 higher than that which is found in receptor rich tissues such as intestine. A minor immunoreactive band is detected above the receptor in cytosols from YEpV1 transformed cells. We believe this is due to reaction with some uncleaved fusion protein and likely is not due to a modification by the host because it is not apparent in cytosol from cells transformed with YEpV3 (Fig. 3A, Lanes 5-8).

Since the CUP1 promoter is tightly regulated by copper ions, it can be used to control expression of receptors in yeast cells. Those vectors containing the CUP1 promoter can be induced to produce receptors by the addition of 100μM of copper sulphate.

Furthermore the CUP1 systems can be used to detect subtle mutations of the receptor which may demonstrate an apparent normal phenotype in an overexpressed state. The inducibility of the system can be seen in Fig. 3B where cells were grown in the absence of copper and in the presence of 5μM and 100μM copper sulphate.

## EXAMPLE 4

### β-Galactosidase and Galactokinase Vectors

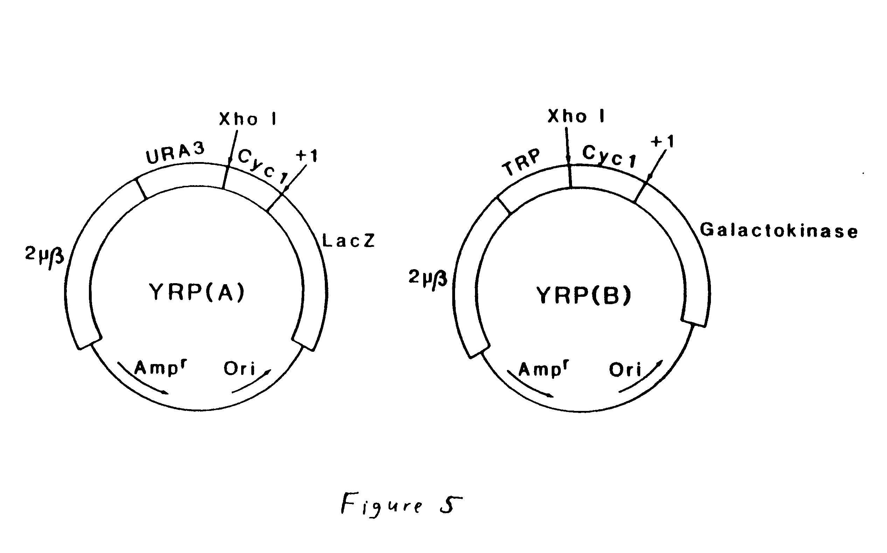

The β-Galactosidase and Galactokinase vectors are shown schematically in figure 5. The YRP(A) and YRP(B) vectors contain an enhancerless promoter and are transcriptionally inactive. YRP(A) and YRP(B) contain a unique XhoI site at -250 where cis elements may be placed to assay enhancer properties. To develop a target plasmid for each of the receptors, specific sequences which have been shown to confer steroid responsiveness upon heterologous promoters were placed into pC2 ($\beta$-Galactosidase) or YCpR1 (Galactokinase) creating reporter plasmids responsive to progesterone, androgen, glucocorticoid, vitamin D and estrogen. The receptors were introduced into cells containing the expression vectors and co-transformants were selected by uracil and tryptophan auxotrophy.

A reporter YRpP1 containing the progesterone responsive sequence and the $\beta$-Galactosidase gene was used to quantitate the transcriptional activity of the chicken progesterone receptor. Yeast containing both the expression and the reporter plasmids was grown overnight in minimal media supplemented with 2% glucose. On the next day these cells were used to inoculate 10 mls of fresh media at a density of 100,000 cells/ml. Four sets of tubes were inoculated (a) no additions, (b) +100$\mu$M CuSO$_4$, (c) +0.1$\mu$M progesterone, and (d) +100$\mu$M CuSO$_4$, 0.1$\mu$M progesterone. When the cells reached mid-log phase they were harvested by centrifugation. The cells were lysed using glass beads and the cytosol was clarified by centrifugation. Aliquots (1-100$\mu$g) of protein were assayed for $\beta$-Galactosidase activity according to the method of Miller and expressed as units/mg protein. The results were as follows :

|  | Control | +CuSO$_4$ | +Prog. | +CuSO$_4$+Prog. |
|---|---|---|---|---|
| Miller units | <500 | <500 | <500 | 50,000 |

In this assay, the progesterone receptor was able to upregulate the expression of the reporter transgene 100-fold. Other data indicate that 1$\mu$M CuSO$_4$ is sufficient to activate half maximally this transcription unit. The ability to regulate the amount of steroid receptor is a major advantage of the CUP promoter over any others. Similar results were observed for the other steroid receptors. For example, YEpV1 for expression of human vitamin D receptor, YEpE2 for expression of human estrogen receptor, YEpAR1 for expression of human androgen receptor, YEpP2 for expression of chicken progesterone receptor ($\beta$ form) YEpP8 for expression of chicken progesterone (A form) ; YEpHPR 1 for expression of human progesterone (A form), YEpHPR2 for expression of human progesterone (B form), YEpHTR1 for expression of thyroid receptor ($\beta$ form), YEpHTR2 for expression of thyroid receptor ($\alpha$ form), YEpRAR for expression of retinoic acid receptor, YEpCOUP for expression of the COUP protein, YEpV9 for expression of chicken vitamin D receptor and YEpV10 for expression of rat vitamin D. The mammalian steroid receptors produced in yeast function identically to those produced in homologous cells. On this basis, two rapid screening protocols were developed to identify receptor agonists and antagonists.

## EXAMPLE 5

### $\beta$-Galactosidase Transcription Assay

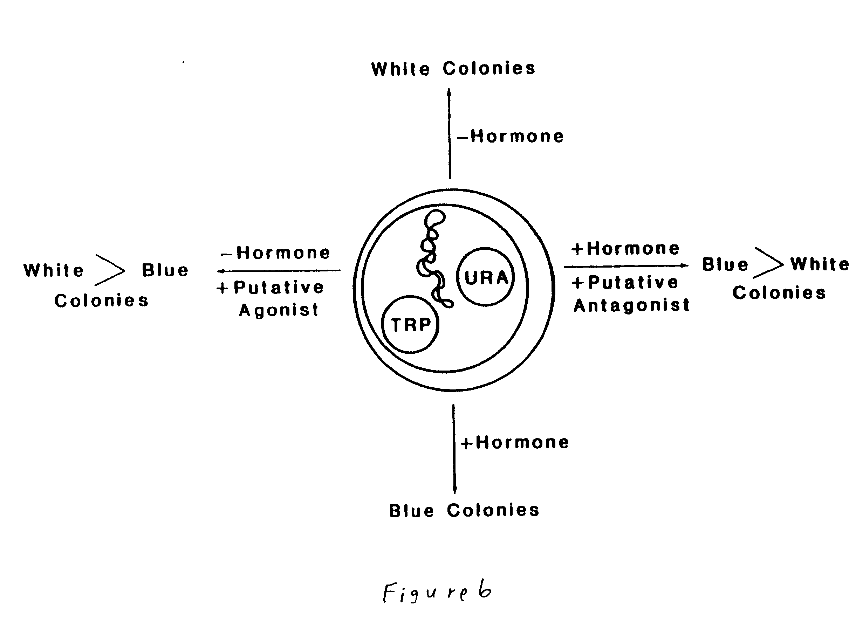

A direct plate method to screen for receptor agonists or antagonists has been developed. The basic assay plates contained selective media (buffered to pH 7.0, X-GAL (5-Bromo-4-Chloro-3-Indolyl $\beta$-D-Galactopyranoside), a $\beta$-Galactosidase substrate, IM CuSO$_4$ and 2% agar as a hardening agent. Other additions to the plates depend on what is being tested. For example, agonist or antagonist is added when appropriate. The strategy for this assay is outlined in Fig. 6. In the absence of any additional factors, colonies containing the receptor and reporter will grow and appear white. If a hormone is present the colonies will appear blue. In the absence of hormone but in the presence of a potential steroid agonist the cells will have a blue appearance, the intensity of which will reflect the agonists efficacy. In the presence of hormone and a putative steroid antagonist, the colonies appear white, the intensity of which reflects the degree of inhibition.

## EXAMPLE 6

### Galactokinase Transcription Assay

An alternative screening strategy is the 2-deoxy-D-galactose/Galactokinase assay. In gal1 strains of yeast, expression of Galactokinase is required for growth. This characteristic can be used as an assay for compounds that act as steroid agonists or may identify mutations of the receptor that effect the receptor in a positive manner. This system also can be used to look for steroid antagonists or to identify mutations that effect receptor function in a negative manner. This is accomplished by growing the strains in media containing a sugar source other than glucose or galactose and including the compound 2-deoxy-D-galactose. The selection is based on the ability of Galactokinase to phosphorylate 2-deoxy-D-galactose and convert it into a cytotoxic compound.

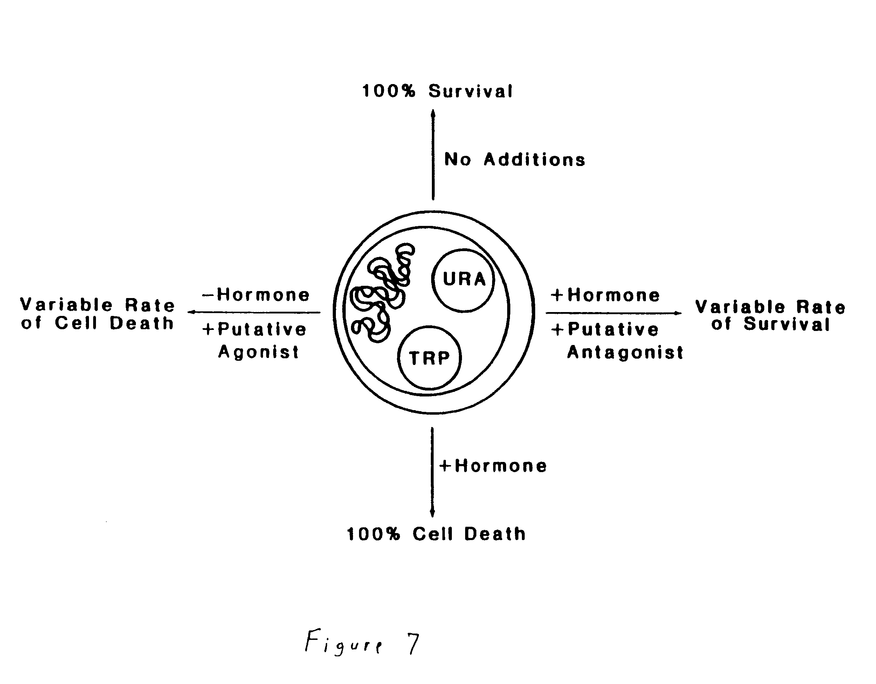

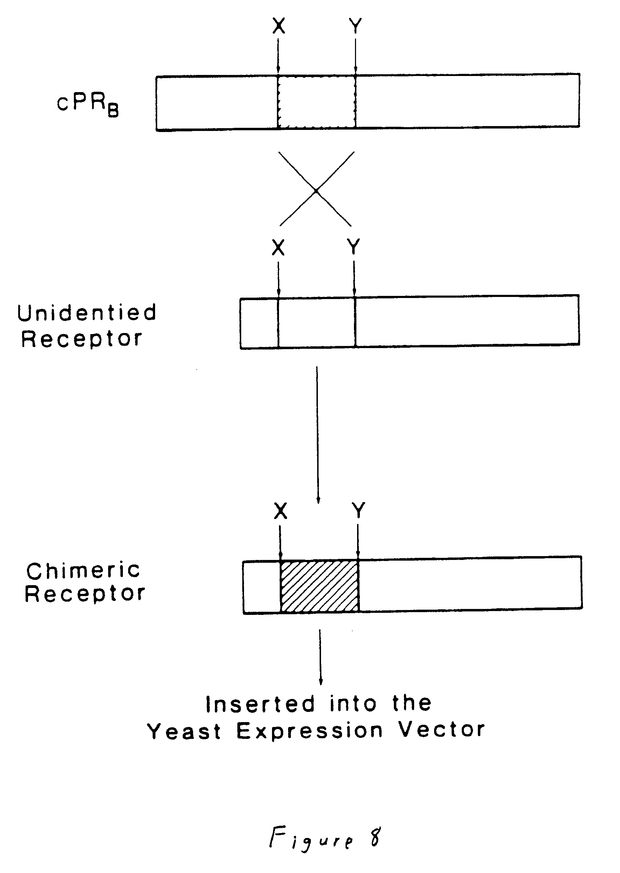

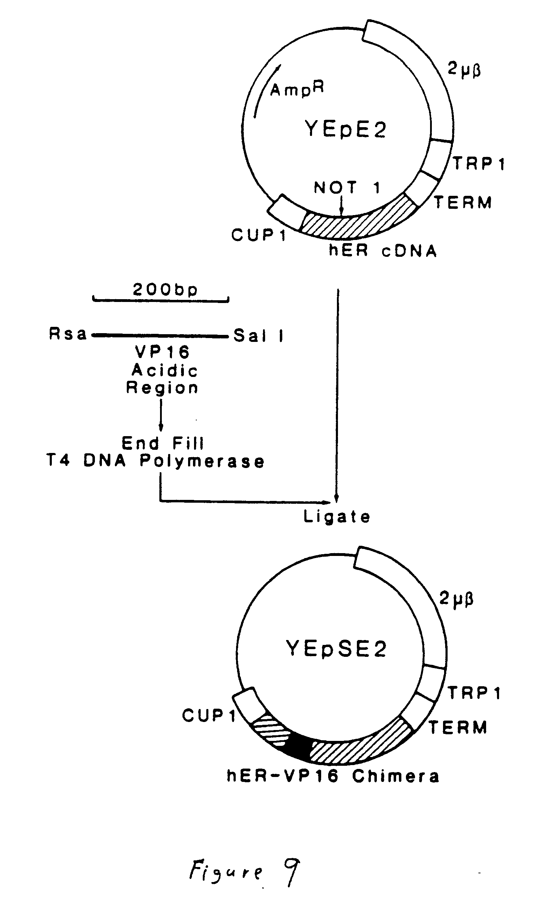

The test plates contained minimal media, 3% glycerol and 2% ethanol as carbon sources, 2% agar, 1μM CuSO₄, and 0.1% 2-deoxy-D-galactose. Other additions depend on the test. For example, agonists and antagonists can be added. The strategy for this screen is outlined in Fig. 7. In the absence of hormone, the cells will grow normally. If hormone is present, the cells will die. In the absence of hormone and in the presence of an agonist, the cells will die. The degree of death will be related to the degree of stimulation by the test compound. In the presence of hormone and in the presence of an antagonist, the cells will survive, and the degree of survival is related to the inhibition properties of the antagonist.

## EXAMPLE 7

### Use of Chimeras to Identify Receptor Ligands

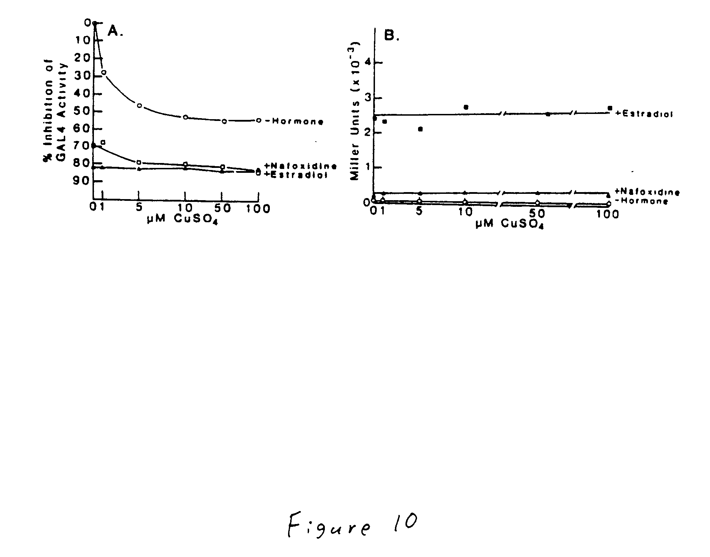

Receptor chimeras comprised of the progesterone receptor and several of the receptor molecules for which a ligand has not been identified have been constructed. A schematic of the strategy to make chimeras is shown in Fig. 8. In one example, specific restriction sites are introduced at the boundaries of the DNA binding domain of the chicken progesterone receptor and compatible sites are introduced into the molecule to be studied. The sequence of the chimera was confirmed across the junction points and the entire cDNA was inserted into the yeast expression vectors as described earlier. These expression plasmids were introduced into cells containing the progesterone reporter plasmids. In the absence of any test compounds, the reporters are not activated. Natural products are screened to identify activators of these chimeras. Fusion of the DNA binding region of the progesterone or estrogen receptor with the hormone binding domain of a receptor with an unknown ligand can be accomplished. The resultant molecule will be used in conjunction with the reporter systems described to screen for molecules that will regulate progesterone responsive reporter genes. The first of these chimeras constructed was the DNA binding region of the progesterone receptor and the carboxyl region of the transcription factor COUP. The COUP factor is a member of the steroid receptor family and is closely related to the estrogen receptor. Receptor chimera's consisting of the DNA binding region of one receptor and the hormone binding of another receptor can be combined to create a functional molecule. The sterol specificity of these chimeras is dictated by the carboxyl terminus.

## EXAMPLE 8

### Conversion of a Human Estrogen Receptor into a Superactive Receptor.

The mechanism by which steroid receptors enhance transcription is unclear, however, there is a clear correlation between the ability of the receptor to activate transcription and the presence of an acidic activation region in the protein. By increasing the number of acidic regions in the receptors we have increased the potency of the receptors. The best results have been obtained by insertion of the acidic activation domain of the herpes simplex VP16 protein into the coding region of the steroid receptors. As illustrated in Fig. 9 this was accomplished for the human estrogen receptor by digestion of the yeast expression vector for estrogen, YEpE2 with Not1, filling in the ends with T4 polymerase and religating in the presence of the filled in Rsal - Sall fragment of the VP16 protein. After the receptor is inserted in the proper orientation it produces a chimeric receptor which includes within its reading frame a VP16 activating domain.

A comparison of the wild type estrogen receptor and the modified receptor was made in yeast using the estrogen responsive reporters. All activities are expressed in Miller units per milligram of protein, E2 is estrodial.

|          | Control | +CuSO₄ | +E2  | +CuSO₄+E2 |
|----------|---------|--------|------|-----------|
| ER       | 447     | 473    | 5760 | 4344      |
| Super ER | <50     | 4133   | 1999 | 23,999    |

The super estrogen receptor clearly elevated the maximum rate of transcription of the reporter over 5-fold. More importantly the background of the system dropped considerably, thereby increasing the sensitivity of the screen. Similar results have been obtained with other steroid receptors, for example YEpV1 for expression of human vitamin D receptor, YEpE2 for expression of human estrogen receptor, YEpAR1 for expression of human androgen receptor, YEpP2 for expression of chicken progesterone receptor, YEpP8 A for expression of chicken progesterone (A form) ; YEpHPR 1 for expression of human progesterone (A form), YEpHPR2 for expression of human progesterone (B form), YEpHTR1 for expression of thyroid receptor (B form), YEpHTR2 for expression of thyroid receptor (α form), YEpRAR for expression of retinoic acid receptor, YEpCOUP for expression of the COUP protein (B form), YEpV9 for expression of chicken vitamin D receptor and YEpV10 for expression of rat vitamin D receptor.

## Example 9

### Improved Clinical Assay for Steroids

Steroids are routinely measured in clinical or laboratory specimens by radioimmunoassay. These assays rely on the ability of antibodies to recognize unique epitopes on the steroids. However for some steroids, for example vitamin D, thyroid hormone and retinoids, the precursors, active metabolities and breakdown products are so antigenically similar that antibodies will not discriminate between these compounds. In these cases it is more accurate to measure steroids by radio-receptor assays. The high specificity of binding of a steroid to its cognate receptor allows the detection of a particular ligand in a complex sample such as serum. This assay consists of pre-labeling a specific receptor with radioactive steroid, and evaluating the ability of radio-inert steroids in a complex mixture to displace the radio-ligand. A standard curve using known amounts of radio-inert steroid allows determination of the exact concentration of ligand in the sample. An example of such an assay is the radio-receptor assay for $1,25(OH)_2D_3$. This ligand is measured by its ability to displace radio-labeled $1,25(OH)_2D_3$ from chicken vitamin D receptor. This receptor is difficult to isolate due to its abundance and is difficult to work with because of its susceptibility to proteases. An improvement to this particular assay is the substitution of chicken intestine receptor with recombinant human vitamin D receptor that is produced in the yeast expression system in the present invention. This receptor is intact and biologically active even when stored at room temperature for at least 1 week. Other examples of radio-receptor assays which can be accomplished using the recombinant receptors of the present invention is the assays for thyroxine or retinoids.

## Example 10

### The use of the reporter systems, steroid receptors, and receptor chimeras to estimate the concentration of ligands in complex samples.

The steroid receptors and receptor chimeras specifically respond to steroid hormones. The specificity of the hormonal response is determined by the carboxyl terminus of the molecule. These response systems can be used to assay the concentration of steroids in a complex specimen. This method has an advantage over radioimmunoassay or radio-receptor assays in that it will only measure biologically active steroid ligands and will specifically discriminate against modified or inactive substrates and intermediate metabolities. One example of this approach is the assay to quantitate estrogen.

Yeast cells containing both the estrogen receptor and a reporter vector are grown overnight in a defined selective media. The next day dilutions of the clinical or laboratory sample are added to yeast cells. Controls can be incubated with known amounts of estrogen. The cells are allowed to sit at 30°C for an additional 4 hours at which time the cells are harvested and the level of β-Galactosidase enzyme produced is measured. A standard curve is constructed using values obtained from the known concentrations of steroid and then by extrapolation from this graph, the concentration of estrogen in the unknown sample is determined.

The efficacy of this method depends on the background and the sensitivity of the system. For this reason some applications of the essay may require the use of the super-estrogen receptors, for example, estrogen receptor with an activation domain of a herpes simplex VP16 protein included within its reading frame.

Because of the modular nature of the receptors, the ligand binding domain of one receptor can be used to replace that of the estrogen receptor. These chimeric receptors will activate reporters containing the estrogen response element in response to addition of the cognate ligand for the ligand binding domain. For example, vitamin $D_3$ can be assayed when expressed to an estrogen receptor chimera containing the C-terminal domain of the vitamin $D_3$ receptor. In this way, it is not necessary to develop a response system for each receptor and chimeric receptors can be used whenever a more sensitive assay is needed.

### EXAMPLE 11

### Construction of plasmids

The expression vector YEpE10 was constructed as follows : The human estrogen receptor plasmid (pGEM35hER) was digested with BamH1 and TTHIII1. A linker which encoded the last 6 amino acids of yeast ubiquitin and also contained an AFL11 and Nco1 site was ligated to the digested plasmid. The EcoR1 site of the resulting plasmid was converted to a Kpn1 site. The AFL11 - Kpn1 fragment of this vector was purified and cloned into the corresponding sites of the yeast expression vector YEpV1 (Example 1), yielding the plasmid YEpE2.

Since the original hER cDNA has been reported to contain a point mutation, this defect was corrected by replacing the ASP718-SmaI fragment of this YEpE2 with the analogous fragment from the wild type cDNA. The resulting plasmid which expresses wild-type hER was called YEpE10. An integrative version of this plasmid was constructed by cloning the BamH1-Mlu1 fragment of this plasmid into the cognate sites of the vector PYSK152. Integration into the yeast genome was accomplished by linearizing the plasmid at a unique BstE11 site within the leu2 sequence and selecting for tryptophan auxotrophs.

Example 12

Construction of Reporter Plasmids

Reporter plasmids were also constructed. The parent vector was PC2. The plasmid was modified by the insertion of a Hind111 and Bg111 site into the unique Xho1 site, thus creating the vector PC3 containing a mini-cloning cassette. Oligonucleotides were constructed with BamH1 ends and inserted into the plasmid PC3 as single and multiple copies. The number of oligonucleotides inserted was determined by restriction analysis and the orientation was confirmed by sequencing. Specifically the sequences of the oligonucleotides used were :

GAL4ERE

GATCAGGAAG ACTCTCCTCC GGTCACAGTG ACC                    33

GAL4HSERE

GATCAGGAAG ACTCTCCTCC GGTCACAGAC TCG                    33

Example 13

Transcription Assays

Cells containing the receptor expression plasmid and the reporter were grown overnight in selective media. This culture was used to inoculate fresh media at a density of 100,000 cells/ml. For experiments requiring activation of GAL4, galactose was used as a carbon source instead of glucose. Estrogen and nafoxidine were added as required at $2 \times 10^{-7}M$ and $2 \times 10^{-6}M$ respectively. Copper sulphate was added at various concentrations to induce synthesis of the estrogen receptor. When the cells had reached an optical density of 1.0 at 600 nm they were harvested and the level of β-galactosidase produced was measured.

Example 14

An in vivo DNA Binding Assay

It is known that the estrogen receptor binds to and activates gene transcription through a 13 bp palindromic sequence (ERE). Placing ERE upstream of the promoter in a CYC-1-β-gal fusion vector and transforming into Saccharomyces cerevisiae results in a reporter which responded to estrogen in a receptor-dependent manner. To monitor the binding of the estrogen receptor to the ERE sequence in vivo, an interference assay was developed. An oligonucleotide containing a GAL4 (17 mer) recognition sequence and an ERE was constructed such that the two sites would overlap by 1 base. In this way only ER or GAL4 but not both molecules could occupy the same site. This sequence was cloned as a single, double, or triple copy distal to the promoter in a yeast iso-l-cytochrome C reporter (PC3).

These plasmids were introduced into yeast and assayed for transcriptional activity in the presence of glucose and galactose. The results are shown in Table I and reveal that galactose induced transcription of the reporter gene, and activity increased dramatically as the number of GAL sites increased. When grown in glucose, transcriptional activity was completely repressed.

### TABLE I ACTIVATION OF GAL4/ERE BINDING SITE CHIMERAS BY GALACTOSE IN BJ3505

| CONSTRUCT | | TRANSCRIPTIONAL ACTIVITY | |
|---|---|---|---|
| Nomenclature | # of Binding Sites | Glucose | Galactose |
| YRpEG1 | 1 | <50 | 860 |
| YRpEG2 | 2 | <50 | 2,350 |
| YRpEG3 | 3 | <50 | 16,500 |

The estrogen responsiveness of these constructs was measured by co-transforming yeast with the reporter plasmid and an expression vector containing the authentic human estrogen receptor under the control of the yeast metallothionein promoter (CUP1). Cells containing these plasmids were grown in glucose in the presence or absence of $10^{-7}$M estradiol. The results are shown in Table II.

### TABLE II ACTIVATION OF GAL4/ERE BINDING SITE CHIMERAS BY ESTROGEN IN B53505/YEpE10

| CONSTRUCT | | TRANSCRIPTIONAL ACTIVITY | |
|---|---|---|---|
| Nomenclature | # of Binding Sites | +Estradiol | −Estradiol |
| YRpEG1 | 1 | 360 | <50 |
| YRpEG2 | 2 | 980 | <50 |
| YRpEG3 | 3 | 2,320 | <50 |

In this case a hormone dependent activation of transcription correlated with the number of GAL4/ERE sites in the plasmid. The reporter YRpEG3 was chosen for further studies since it distinguished the transcriptional activities resulting from GAL4 and estrogen receptor.

Example 15

Interaction of the Estrogen Receptor with DNA is Hormone Dependent

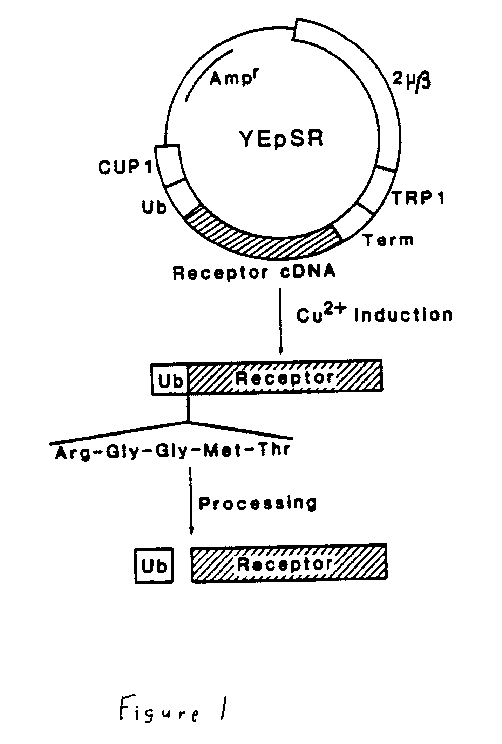

The interference assay of Example 14 was employed to monitor binding of estrogen receptor to its cognate ERE in the absence of hormone. For this experiment cells containing a high copy estrogen receptor expression plasmid and YRpEG3 (contains three copies of GAL4/ERE chimeric response element) were grown in galactose in the presence or absence of $10^{-7}M$ estradiol and receptor levels were induced by the addition of increasing concentrations of copper sulfate to the media (0-100uM). Results are shown in Figure 1A. In the absence of hormone at low receptor concentrations, the GAL4 reporter demonstrated a high transcriptional activity (24,000 miller units, 0% inhibition). This level decreased to 50% of original upon generation of increasing concentrations of cellular receptor by copper addition. Hormone binding analysis demonstrated that the receptor concentration under these conditions increased 5-fold from 0.22pMoles/mg protein in the absence of copper to 1.28 pMoles/mg in the presence of 100uM $CuSO_4$.

At low receptor concentrations (<1 µM $CuSO_4$), GAL4 activity was reduced by approximately 80% when the cells were grown in the presence of estradiol. Thus, at low concentrations of receptor the molecules were maintained in an inhibitory complex in the absence of hormone ; the inhibition can be exceeded in part at higher levels of receptor expression. An analogous result was observed when the experiments were carried out in the presence of nafoxidine, a known anti-estrogen. Addition of this anti-estrogen resulted in a similar inhibition of GAL4 activity, implying that it provided binding of the estrogen receptor to response element. Thus, the mechanism of action of this anti-estrogen must involve a step subsequent to receptor transformation to a DNA binding form. The final level of repression of GAL4 activity observed for each of these ligands represents a balance between activation by the estrogen receptor and interference of GAL4 activity.

Example 16

Measurement of Receptor Transcriptional Activity

The transcriptional activity of the receptor was assayed under similar conditions to Example 15 except that the cells were grown in glucose (Fig. 1B). In the presence of glucose, the transcriptional activity of GAL4 is completely repressed because the GAL4 protein is incapable of binding to its response element. Under these conditions estrogen receptor activation of the GAL4-ERE can be monitored without interference. Maximal activation of the ERE element was observed at the lowest concentration of $CuSO_4$. At all concentrations of receptor the activation of transcription was entirely hormone dependent. Under these conditions, the partial agonist activity of nafoxidine was about 20% that of estrogen. Further, copper, estrogen or nafoxidine had no direct effects on the activity of the YRpEG3 reporter alone.

Example 17

Sensitivity of GAL4-ERE Assay

GAL4 is a potent transactivator that is present in yeast in low concentrations. Therefore, to show that the assay reflects the behavior of the majority of the molecules and that under activating conditions a small amount of receptor activity does not distort the assay, the expression vector YIpE10 was constructed. This vector integrates into the genome as a single copy and produces only 10% of the receptor observed using the high copy expression vector. Hormone binding analysis indicated that the concentration of estrogen receptor in the absence of copper is 25 fmol/mg protein and in the presence of 100uM CuSO4 it increases to 115fmoles/mg protein.

The reporter YRpEG3 was introduced into the yeast strain containing the integrated receptor expression vector and the transcriptional activity was examined following growth of the cells in glucose and galactose. The results are shown in Fig. 2. In galactose, (Fig. 2A), a 30% reduction of GAL4 mediated transcription was seen when the cells were grown in the presence of increasing concentrations of copper but in the absence of hormone. At the lowest concentration of receptor, in the presence of either estradiol or nafoxidine, the GAL4 activity was reduced by approximately 90%. In the case of estrogen mediated inhibition, the lowest concentration of receptor was again effective. The reason that increasing the receptor concentrations had no further inhibitory effect was most probably due to compensatory activation of transcription mediated by estrogen receptor (Fig. 2B). In the presence of nafoxidine, however, increasing the concentration of receptor reduced the GAL4 transcriptional activity by 97% and reflects the much weaker agonist activity of nafoxidine.

To show that the receptor was in fact limiting, cells were grown in glucose. It was observed that even at 100uM $CuSO_4$, estrogen mediated activation of the reporter was sub-maximal. Under these conditions activation of reporter transcription by nafoxidine was detected. Thus, estradiol mediated activation requires only low levels of receptor and illustrates the sensitivity of the GAL4-ERE assay. This sensitivity is likely to be a result of the requirement that all three response elements must be occupied for maximal activation of YRpEG3 in galactose (Fig. 2B). However, because of the cooperative nature of the GAL4 binding, occupancy of a single site can theoretically reduce activity by 85% (Table 1 and Fig. 2A).

## Example 18

Hormone Induced Binding of Estrogen Receptor at Physiological Concentrations of Estrogen

In order to demonstrate that the GAL4 inhibition assay in yeast reports on a bona fide hormone response, the experiments were repeated at different concentrations of estrogen using cells containing the expression plasmid YIpE10. The results are shown in Figure 3. The amount of hormone required for half-maximal inhibition of transcription is analogous to what is required for activation. This argues for and confirms a hormone and receptor dependent interference in GAL4 transcriptional activity against a secondary event being responsible for the observed inhibition.

## Example 19

Inhibition of GAL4 Activity Requires a Functional Estrogen Response Element

The following example shows that the transcriptional interference observed was due to a direct interaction of the hormone receptor complex with DNA and was not the result of squelching of some transcription factor required for GAL4 function. In this example an ERE oligonucleotide was generated that contained just one estrogen response element half site, the second half site being replaced by random sequence of the same composition. This oligonucleotide binds the estrogen receptor with a lower affinity. Four copies of this oligonucleotide were inserted into the vector PC3 and the resulting recombinant (YRpHSEG4) was transformed into yeast cells containing the integrated estrogen receptor expression vector. The results are shown in Figure 4.

This reporter demonstrates the usual high transcriptional activity in the presence of galactose. Increasing the receptor concentration in the absence of hormone reduced the activity of the reporter by 18%. With this reporter, the ability of the ER :nafoxidine complex to inhibit GAL4 activity was less than that of ER :estradiol complex. This result was observed consistently, and may reflect a qualitative difference in these two receptor-DNA complexes.

The results reflect the reduced affinity of the estrogen receptor for this mutated ERE. When this reporter was analyzed in glucose a 2-3 fold activation of transcription was observed. This demonstrated that in vivo the estrogen receptor is capable of interacting with the receptor half-site. Clearly, however, the activity of the reporter was not compromised by the hormone activated receptor to the same extent as when the full estrogen response element was employed (Fig. 3A). Taken together, the data demonstrates that the hormone dependent inhibition of GAL4 activity observed in the assay of the present invention is due to ligand dependent binding of the estrogen receptor to its ERE and that squelching does not play a significant role.

One skilled in the art will readily appreciate that the present invention is well adapted to carry out the objects and obtain the ends and advantages mentioned as well as those inherent therein. The molecules, vectors, reporters, plasmids, chimeras, products, methods, assays, procedures and techniques described herein are presently representative of the preferred embodiments, are intended to be exemplary and are not intended as limitations on the scope. Changes therein and other uses will occur to those skilled in the art which are encompassed within the spirit of the invention or defined by the scope of the pending claims.

## Claims

1. A method for making an expression vector which produces a biologically active receptor, comprising the steps of :
   synthesizing a linker having the carboxyl 6 amino acids of ubiquitin and an internal restriction site ;
   cloning said synthesized linker to form a plasmid ;
   digesting said plasmid with a restriction endonuclease compatible with said internal restriction site;
   inserting a cDNA coding for a receptor into the compatible restriction site of said plasmid ; and
   transforming yeast with said receptor cDNA plasmid.

2. The method of claim 1, wherein the yeast is selected from the group consisting of Saccharomyce and Aspergillus.

3. A transformed yeast made by the method of claim 1, wherein the receptor cDNA is selected from the group of cDNA coding for estrogen receptor, androgen receptor, vitamin D receptor and progesterone receptor.

4. An expression vector YEpV1 for expression of human vitamin D receptor.

5. An expression vector YEpE2 for expression of human estrogen receptor.

6. An expression vector YEpARI for expression of human androgen receptor.

7. An expression vector YEpP2 for expression of chicken progesterone receptor. (B form)

8. An expression vector YEpP8 for expression of chicken progesterone receptor. (A form)

9. An expression vector YEpHPR1 for expression of human progesterone (A form).

10. An expression vector YEpHPR2 for expression of human progesterone (B form).

11. An expression vector YEpHTR1 for expression of thyroid receptor (B form).

12. An expression vector YEpHTR1 for expression of thyroid receptor ($\alpha$ form).

13. An expression vector YEpRAR for expression of retinoic acid receptor.

14. An expression vector YEpCOUP for expression of COUP protein.

15. An expression vector YEpV9 for expression of chicken vitamin D receptor.

16. An expression vector YEpV10 for expression of rat vitamin D receptor.

17. A reporter vector, comprising :
    a proximal promoter element of yeast iso-l-cytochrome C ; and
    a structural gene fused to said promoter, wherein said structural gene is selected from the group consisting of $\beta$-Galactosidase, Galactokinase and URA3.

18. The reporter vector of claim 17, further comprising an insertion site for a receptor response element.

19. The reporter vector of claim 18, further comprising a receptor response element.

20. The reporter vector of claim 19, wherein the receptor response element is selected from the group consisting of progesterone receptor response sequence, androgen receptor response sequence, glucocorticoid receptor response sequence, vitamin D receptor response sequence, estrogen receptor response sequence, thyroid receptor response sequence and retinoic acid receptor response sequence.

21. A reporter vector YRP(A).

22. A reporter vector YRP(B).

23. A receptor agonist or antagonist transcription assay, comprising the steps of :
    growing the reporter vector of claim 19 in appropriate media ;
    adding test agonists or antagonists ; and
    examining the resultant growth.

24. The assay of claim 23, wherein the receptor vector includes the $\beta$-Galactosidase as the structural gene ; and the media includes 5-bromo-4-cholro-3-indolyl $\beta$-D-galactopyranoside, $\beta$-Galactosidase substrate, $CuSO_4$ and agar.

25. The assay of claim 23, wherein the receptor vector includes Galactokinase as the structural gene ; and the media includes 3% glycerol, 2% ethanol, 2% agar, $CuSO_4$ and 0.1% 2-Deoxy-D-Galactose.

26. A method of making a chimera receptor comprising the steps of :
    excising with a restriction endonuclease a DNA binding domain from a known receptor ;

introducing a compatible restriction site at a DNA binding domain in an unknown receptor ;

excising the DNA binding domain from the unknown receptor at said compatible restriction site ; and

inserting into said unknown receptor at the compatible restriction site the known DNA binding domain.

27. An assay for the identification of an unknown receptor comprising the steps of :

growing a chimera receptor made by the method of claim 26 ;

adding appropriate agonists to screen the unknown portion of the chimera receptor ; and

measuring the responsive of the known DNA receptor binding domain.

28. An assay to measure a biological compound in a biological specimen comprising the steps of :

pre-labeling a biologically active receptor with an appropriate labeled biological compound, wherein said receptor is synthesized by the expression vector of claim 1 ;

adding a sample of said specimen to the pre-labeled receptor ; and

measuring the amount of labeled biological compound displaced by the biological compound in said specimen to determine the amount of biological compound in said specimen.

29. The assay of claim 28, wherein the label is selected from the group consisting of radioisotopes, fluorescers, chemiluminescers, enzymes and antibodies.

30. The assay of claim 29, wherein the label is a radioisotope.

31. The assay of claim 28, wherein the biologically active receptor is selected from the group consisting of estrogen receptor, androgen receptor, vitamin D receptor, progesterone receptor, cortisone receptor, prednisone receptor, prednisolone receptor, aldosterone receptor, retinoic acid receptor and thyroid receptor.

32. A kit for measuring a biological compound in a biological specimen, comprising, a container having a biologically active receptor synthesized by the expression vector of claim 1.

33. The kit of claim 32, further comprising, a radio-labeled compound for binding to the biologically active receptor.

34. The kit of claim 32, further comprising control samples to generate a standard curve.

35. An assay for measuring a biological compound in a biological specimen comprising the steps of :

growing yeast cells containing a biologically active receptor and a reporter vector, wherein the reporter vector comprises a proximal promoter element of yeast iso-l-cytochrome fused to a structural gene selected from the group consisting of β-Galactosidase, Galactokinase and URA3 ;

adding a sample of said specimen to the yeast cells ; and

determining the amount of biological compound in said specimen by measuring the activity of the protein product of the structural gene.

36. The assay of claim 35, wherein the receptor is a chimeric receptor.

37. The assay of claim 36, wherein the chimeric receptor includes the estrogen response element and a non-estrogen ligand binding domain.

38. The assay of claim 37, wherein the ligand binding domain is selected from the group consisting of estrogen receptor, androgen receptor, vitamin D receptor, progesterone receptor, cortisone receptor, prednisone receptor, prednisolone receptor, aldosterone receptor, retinoic acid receptor and thyroid receptor.

39. The assay of claim 35, wherein the receptor is a superactive receptor.

40. The assay of claim 39, wherein the superactive receptor is the estrogen receptor with an activation domain of a herpes simplex VP16 protein included within its reading frame.

41. The assay of claim 35, wherein the receptor is selected from the group consisting of estrogen receptor, androgen receptor, vitamin D receptor, progesterone receptor, cortisone receptor, prednisone receptor, prednisolone receptor, aldosterone receptor, retinoic acid receptor and thyroid receptor.

**42.** The assay of claim 41, wherein the receptor is an estrogen receptor and the structural gene is β-Galactosidase.

**43.** A reporter YRpHSEREG4.

**44.** A reporter YRpEG1

**45.** A reporter YRpEG2

**46.** A reporter YRpEG3

**47.** A reporter for detecting DNA binding, comprising a sequence for any transcription factor that binds to DNA and a sequence for a response element from any biologically active receptor ; wherein the sequence for the transcription factor and the sequence for the response element are fused such that only one protein can occupy the fused sequence at any given time.

**48.** The reporter of claim 47 wherein the transcription factor and response element sequences are fused such that at least one base overlaps.

**49.** The reporter of claim 47 wherein the transcription factgor is selected from the group consisting of GAL4, GCN4, yAP1, and ACE1 ; and the response element is selected from the group consisting of recognition sequences for estrogen receptor, androgen receptor, vitamin D receptor, progesterone receptor, cortisone receptor, prednisone receptor, prednisolone receptor, aldosterone receptor, retinoic acid receptor and thyroid receptor.

**50.** The reporter of claim 47 wherein the transcription factor is GAL4 and the response element is estrogen receptor recognition sequence.

**51.** The reporter of claim 47 wherein there are a plurality of fused sequences.

**52.** An assay for measuring a biological compound in a biological specimen which binds to the active receptor of the reporter of claim 47, comprising the steps of :
inserting the reporter and a biologically active receptor into a cell ;
growing the cell in appropriate media ;
adding the biological specimen to the growing cells ;
measuring the amount of transcriptional activity, wherein said transcriptional activity is proportional to the amount of biological compound in said biological specimen.

**53.** The assay of claim 52 wherein the cells are selected from the group consisting of Saccharomyce and Aspergillus.

**54.** An assay to screen for agonists or antagonists comprising the steps of :
inserting the reporter of claim 47 and a biologically active receptor into a cell ;
growing the cell in appropriate media ;
adding test agonists or antagonists ; and
examining the resultant transcriptional activity.

**55.** The method of identifying unknown transcription factor comprising the steps of :
inserting into a cell a reporter of claim 47 and a known biologically active receptor, wherein the reporter includes the unknown transcription factor fused to a known response element ;
growing the cell in appropriate media ;
adding the compound that binds to the biologically active receptor ;
adding test protein to identify the transcription factor ; and
examining the resultant transcriptional activity.

**56.** The method of identifying unknown receptor comprising the steps of :

inserting into a cell a reporter of claim 47 and the unknown receptor, wherein the reporter includes a known transcription factor and the response element of the unknown receptor ;

growing the cell in appropriate media ;

adding a test compound to identify the biologically active receptor ; and

examining the resultant transcriptional activity.

Figure 1

Strategy B:

Figure 2

EP 0 441 483 A2

Figure 3

**DNA Cellulose Chromatography of
Yeast-Derived Human Vitamin D Receptor**

Figure 4

Figure 5

Figure 6

EP 0 441 483 A2

100% Survival

No Additions

Variable Rate
of Cell Death

−Hormone

+Putative
Agonist

URA

TRP

+Hormone

+Putative
Antagonist

Variable Rate
of Survival

+Hormone

100% Cell Death

Figure 7

X          Y

cPR$_B$

Unidentied
Receptor

X          Y

Chimeric
Receptor

X          Y

Inserted into the
Yeast Expression Vector

Figure 8

Figure 9

Figure 10

Figure 11

Figure 12

Figure 13